# EUROPEAN PATENT SPECIFICATION

(11) **EP 2 245 569 B1**
(45) Date of publication and mention of the grant of the patent: **12.08.2020**
(21) Application number: 08789145.3
(22) Date of filing: 29.02.2008
(51) Int. Cl.: G16H 30/20, G16H 50/50

(54) **MEDICAL IMAGING METHOD AND SYSTEM FOR PROVIDING A FINITE-ELEMENT MODEL**
MEDIZINISCHES ABBILDUNGSVERFAHREN UND SYSTEM ZUR BEREITSTELLUNG EINES FINITE-ELEMENTE-MODELLS
PROCÉDÉ D'IMAGERIE MÉDICALE ET SYSTÈME DESTINÉ À FOURNIR UN MODÈLE D'ÉLÉMENT FINI

(43) Date of publication of application: 03.11.2010
(73) Proprietor: Ecole Nationale Supérieure d'Arts et Métiers (ENSAM), 75013 Paris (FR); Centre National de la Recherche Scientifique (C.N.R.S.), 75794 Paris Cedex 16 (FR)
(72) Inventor: SKALLI, Wafa, F-75013 Paris (FR); MITTON, David, F-94270 Kremlin Bicetre (FR); DUBOUSSET, Jean, F-75013 Paris (FR); LAVASTE, François, F-91240 Saint Michel Sur Orge (FR)
(74) Representative: Plasseraud IP
(86) International application number: PCT/IB2008/051807
(87) International publication number: WO 2009/106937

(56) References cited:
- US-A- 5 172 695
- YU ET AL.: "Effect of the Plantar Fasciotomy on the Movement of the Foot Arch" PROCEEDINGS OF ICBBE 2007, 16 July 2007 (2007-07-16), pages 478-481, XP002503357
- WADA ET AL.: "Relationships among bone mineral densities, static alignment and dynamic load in patients with medial compartment knee osteoarthritis" RHEUMATOLOGY, vol. 40, no. 5, 5 May 2001 (2001-05-05), pages 499-505, XP002503358
- PAUL: "Force Actions Transmitted by Joints in the Human Body" PROCEEDINGS OF THE ROYAL SOCIETY OF LONDON, vol. 192, no. 1107, 1976, page 163172, XP002503359
- EKENSTAM ET AL.: "The load on the radius and ulna in different positions of the wrist and forearm: A cadaver study" ACTA ORTHOPAEDICA, vol. 55, no. 3, 1984, pages 363-365, XP002503360

## Description

### FIELD OF THE INVENTION

The instant invention relates to medical imaging methods and systems for providing a finite-element model.

### BACKGROUND OF THE INVENTION

When it comes to mechanical structures, the advantages of the finite-element-method are well known. Recently, attempts were made to use this method to simulate the behaviour of body structures, in particular bony structures of a patient as a mechanical system. Such simulations are difficult: it is necessary to obtain a precise geometry of the bony structure, the mechanical properties of the internal body organs, and the applied loads (also called "boundary conditions"). An attempt to provide such a finite-element-model is described in US 5,172,695. In this patent, the three-dimensional geometry is obtained using a conventional Computer Tomography scanner. The volumetric image provided by the scanner is also a way to quantify the local mechanical properties, by linking the grey value of the voxel to the bone mineral density, which itself can be correlated to the mechanical characteristics of the bone. Then, a finite-element calculation is performed by applying loads on a bony surface.

There is a need to improve the clinical relevance of such calculations, so that their results could be used by clinicians.

US 5172695 discloses a method bone structure data being patient specific so as to determine structure patient specific mechanical characteristics.

### SUMMARY OF THE INVENTION

To this aim, according to the invention, a method according to claim 1 is provided.

With these features, it is possible to take into account the position of the patient during the acquisition, so that the applied load used for the calculation corresponds to the one actually applied on the bony structure in this functional position.

In some embodiments, one might also use one or more of the features defined in the dependent claims. Advantages of some of these embodiments include reduction of the radiation dose applied to the patient.

### BRIEF DESCRIPTION OF THE DRAWINGS

Other characteristics and advantages of the invention will readily appear from the following description of two of its embodiments, provided as a non-limitative example, and of the accompanying drawings.

On the drawings :
- Fig. 1 is a perspective view of an example of a medical imager according to an embodiment of the invention,
- Fig. 2 is a schematic view of the computerized system of Fig. 1,
- Fig. 3 is a perspective view of an example of a generic model for use in a method according to an embodiment of the invention,
- Fig. 4 is a schematic view of a computer screen on which detection data is schematically represented,
- Fig. 5 is a schematic view of an FEM model,
- Fig. 6a and 6b are schematic views with a basic muscular model, illustrating how the loads can be estimated using the patient position-specificity,
- Fig. 7 is a cross-sectional view through a patient, showing a plurality of applied muscular forces illustrating data related to a more sophisticated muscular model,
- Fig. 8 is a schematic view of a result of a FEM calculation for a FEM model such as the one of Fig. 5, and
- Fig. 9 is a schematic perspective view of another possible embodiment for an installation.

On the different figures, the same reference signs designate like or similar elements.

### DETAILED DESCRIPTION

The scope of the invention is defined by the appended claims. Other embodiments herein decribed have to be interpreted as examples.

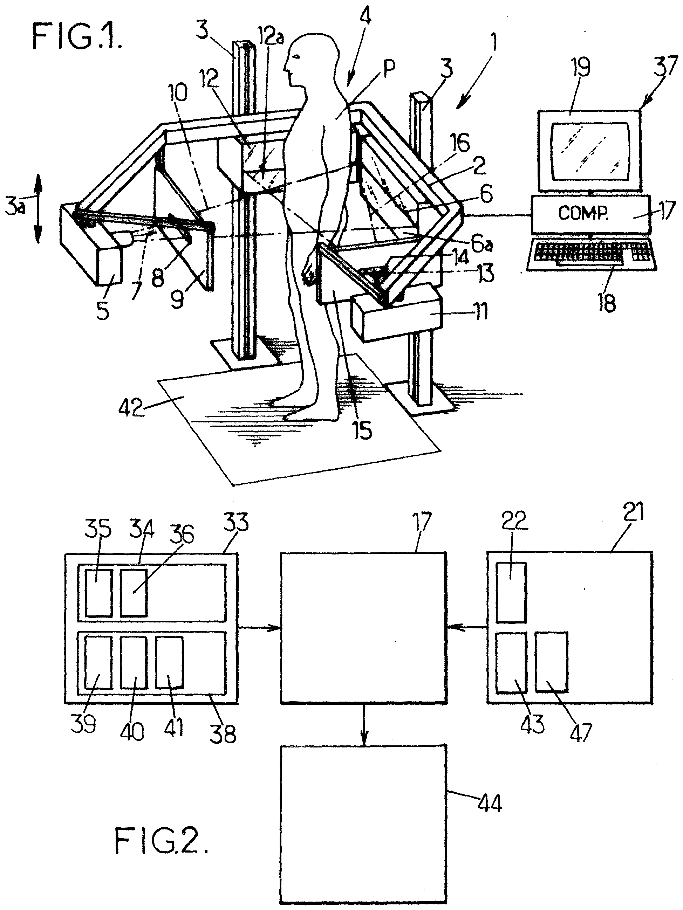

Figure 1 shows a radiographic apparatus 1 for three-dimensional reconstruction, the apparatus comprising a moving frame 2 displaceable under motor drive along vertical guides 3 in both directions of translation 3a.

The frame surrounds a field of observation 4 in which a patient P may be placed in a given position, e.g. standing, for observing an osteo-articular structure of the patient when in the standing position, which may be relevant for patients suffering from postural imbalance for example.

The moving frame 2 carries a first radiological source 5 and a first detector 6 which is placed facing the source 5 beyond the field 4, and which comprises at least one horizontal line 6a of detector cells. By way of example, the detector 6 may be a gas detector responsive to low doses of radiation, e.g. as described in documents FR-A-2 749 402 or FR-A-2 754 068. Naturally, other types of detectors may optionally be used in the context of the present invention.

The radiological source 5 is adapted to emit ionizing radiation, in particular X-rays, suitable for being detected by the detector 6 in an image-taking direction 7 that is antero-posterior relative to the patient P, the rays passing through a horizontal slit 8 made through an aiming mask 9 such as a metal plate in order to generate a horizontal beam 10 of ionizing radiation in the field of observation 4.

The moving frame 2 also carries a second radiological source 11 similar to the source 5 and a second detector 12 similar to the detector 6, disposed facing the source 11 beyond the field 4, and comprising at least one horizontal line 12a of detector cells.

The radiological source 11 is adapted to emit ionizing radiation in a image-taking direction 13 that is lateral relative to the patient P, passing through a horizontal slit 14 formed in an aiming mask 15 such as a metal plate in order to generate a horizontal beam 16 of ionizing radiation in the field of observation 4.

Naturally, there could be more than two radiological sources and detectors, and the image-taking directions of these various radiological sources could, where appropriate, be other than mutually perpendicular, and they need not even be horizontal.

One or each source 5, 11 can be made to emit a beam of X-rays distributed on two different energy peaks, for example by the use of different filters. For example, the source 5 emits a beam of low energy and a beam of high energy alternately during the vertical scanning. The obtained image carries both high energy and low energy acquisition.

In the present example, the patient P is standing on a force-platform 42 which enables to provide the weight and the position of the gravity line of the patient. In another embodiment, the weight data of the patient could be measured by a separate device, before the patient steps into the radiographic apparatus.

The two detectors 6, 12 are connected to a computerized system 37 or some other electronic control system provided with:
· an input interface comprising at least a keyboard 18 and generally also a mouse (not shown);
· an output interface comprising at least a screen 19; and
· a processor 17, for executing a computer program adapted to implement the method described herein.

The computerized system 37 may also be connected to the motor-driven drive means (not shown) contained in the guide 3, and to the sources 5 and 11, so as to control the vertical displacement of the frame and/or the emission of ionizing radiation.

By way of example, the method which is described below is a method of obtention of a finite-element model of a vertebra using a knowledge base of vertebrae. However, this method could be used for any bone or osteo-articular structure of the body such as for example, the femur, the upper limb, the lower limb, the hip, or even part or totality of the skeleton, when a knowledge base of the structure to be reconstructed is provided.

As an example shown on Fig. 2, the processor 17 is connected to a detection memory 33 comprising detection data of the patient P.

The detection data of the patient P comprises detection data 34 of the bone structure of the patient, such as, for example, antero-posterior 35 and lateral 36 (possibly dual-energy) X-ray images of the patient taken with the apparatus 1. In some embodiments, the detection data can further comprise external detection data 38 of the patient such as, for example, antero-posterior 39 and lateral 40 detection of the external envelope (skin) of the patient detected on the antero-posterior and lateral radiographs, respectively, and/or, for example, data of the 3D envelope of the patient and/or weight data 41 of the patient, for example provided from the force platform 42.

The processor 17 is further connected to a knowledge base memory 21 comprising a knowledge base 22 of the geometry of the bone structure, a knowledge base 43 related to the mass and the location of the center of gravity of patient body segments with regard to the bone(s) of interest, and a knowledge base 47 of bone mechanical characteristics. There are multiple ways in which the knowledge can be stored in the knowledge base(s).

As will be described in more details below, the processor 17 calculates, from the detection data and the knowledge base a patient-specific position-specific finite-element model (FEM) which can be used in an appropriate FEM-solver 44. Such FEM-solvers are commercially available and will not be described in more details.

The detection memory 33, the processor 17, the knowledge base memory 21 and the FEM-solver 44 could be found on the same computerized system, or distributed over a network.

For the bone structure under study, the knowledge database may be constructed as or from data obtained from similar structures.

The geometry knowledge database 22 is arranged so as to store surface data relating to coordinates of points belonging to a surface of a generic model.

In this example, the surface data comprise information about points corresponding to particular reference marks on structures of the same type of the studied structure, acquired beforehand, for example by computer tomography.

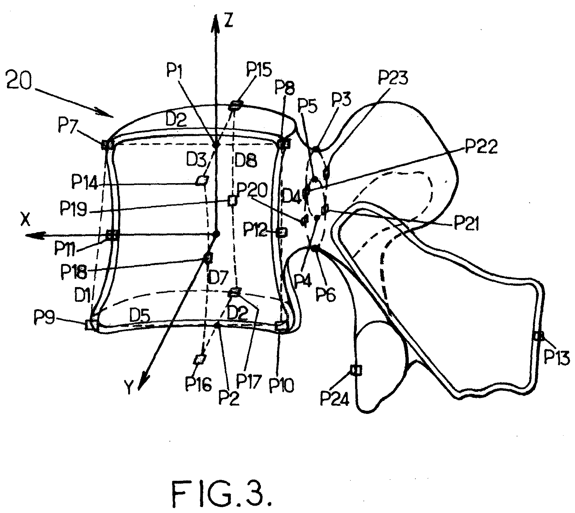

By way of example, the geometry knowledge database 22 of the vertebra contains the coordinates x_{P1}, y_{P1}, Z_{P1},..., x_{P23}, y_{P23}, Zₚ₂₃, of characteristic points P1,..,P23 for the vertebra of each of a plurality of previous patients, characteristic lengths D1..D8 for each vertebra, as shown in Fig. 3, segments, straight lines or arcs that are characteristics of the object, and/or outlines and edges of these particular vertebra.

As shown in Fig. 3, the coordinates of characteristic points or lines may be expressed, for example, in a local X, Y, Z frame of reference.

Further, the outer and inner surface data may further comprise a mesh of several hundred to several hundred thousand points and/or elements of an average vertebra.

It is possible to establish a subset (not represented) of the knowledge database related to vertebrae belonging to healthy individuals or to individuals suffering from different pathologies, and similarly it is possible to characterize each vertebral mesh as a function of the weight, the size, the age, or any other type of parameter concerning the individual that is deemed to be necessary.

Alternatively, in a not represented embodiment, the vertebral mesh may be obtained from a mathematical model constructed from the previously acquired data. By way of example, the surface data may include statistical data (means, variances...) for each parameter of the knowledge base. The surface data may include mathematical equations for determining from the knowledge base of a given vertebra, the positions of the characteristic points for a personalised mesh on the basis of values of estimator parameters for said object. For example, the coordinates of the characteristic points may be parameterized by functions of these parameters.

As used herein, the term "data" represents as well raw data such as obtained from measurements of similar structures, and elaborated data such as equations or the like obtained from the observation of such measurements. The word "data" is used to represent any knowledge which can be stored on a medium and relate to the studied structure.

The mechanical characteristic knowledge data base 47 can for example have been previously obtained from imaging samples from anatomical specimen by X-rays, so as to determine the grey-level of the sample (the bone's mineral density), then by determining in vitro the bone's mechanical properties by mechanical testing (compression, traction and/or bending tests), so as to correlate the grey level and the mechanical characteristics of the sample.

Referring back to Fig. 1, the computer 37 is used initially to take two radiographic images of the patient P by causing the field of observation 4 to be scanned by the beams 10 and 16 of ionizing radiation over a height corresponding to the structure of the patient that is to be observed, for example the spine and the pelvis, or indeed the entire skeleton. For this purpose, the frame is displaceable for example over the height of the patient.

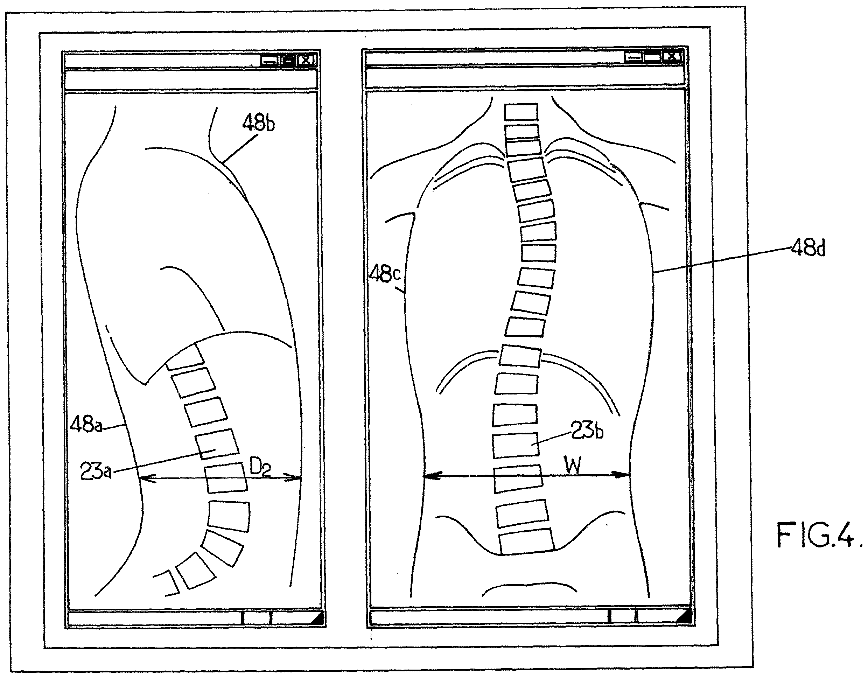

During this movement, two calibrated digital radiographic images of the portion of the patient under examination are stored in the memory 33 of the microcomputer 37, for example an antero-posterior image and a lateral image respectively, wherein each images can be viewed on the screen 19 of the microcomputer, as shown schematically in Figure 4. Of course, Fig. 4 is just an illustrative schematic representation of radiographs, which are grey-levelled images.

The processor 17 receives two-dimensional patient-specific detection data 35, 36, i.e. the two radiographic images of the patient P. The processor 17 comprises or communicates with the memory 21 that stores the knowledge database of the structure 22 shown in Fig.2.

The processor selects an initial model in the geometry knowledge database 22 of the bone from the detection data 35, 36. The selection step may take into account parameters such as a previous radiography of a same patient, a knowledge of the part of the patient that is being imaged etc. to identify an initial model.

In an alternative embodiment, the selection step is performed by an operator, who selects an initial model, and might also point out parts 23a, 23b of the images corresponding to the model.

In an alternative embodiment, the method is to be used for imaging a single type of structure, e.g. femurs, and the base 22 may contain data relative to a single model, e.g. of an healthy femur. In this case, the selection step is trivial.

The invention is by no means limited by the implementation of the selection step.

The 3D outer surfacic model could be obtained by already known methods such as the one described in Laporte et al. "A Biplanar Reconstruction Method Based on 2D and 3D Contours: Application to the Distal Femur", Computer Methods in Biomechanics and Biomedical Engineering 6(1), 1-6, 2003.

Although this article is related to the distal femur, the method described there could be used for any other bone structure. This exemplary method could be summarized as follows :
- the initial model is placed in virtual space so as to roughly correspond to the images of the structure,
- anatomical regions are defined on the generic model,
- 2D contours Λⱼ are identified on the radiographs,
- 2D contours Ωⱼ in the plane of the radiographs are generated from the initial model,
- contours Ωⱼ and Λⱼ are associated to one another,
- the initial model is deformed (for example by combination of a rigid transformation and an homothetic transformation) and contours Ωⱼ are calculated for the deformed model, until contours Ωⱼ and Λⱼ fit,
- the deformed model is further deformed so as to be optimized, for example by kriging.

A three-dimensional position-specific patient-specific surfacic geometric model of the bone structure of the patient is thereby obtained in the frame of reference of the imager. This model is represented as a mesh comprising, for example, surfacic shell elements 46, for representing the cortical bone. For example, the inside volume can be meshed with internal (not shown) volumic elements, for representing the trabecular bone. In addition, the model is obtained in the frame of reference of the imager, i.e. corresponds to the actual position of the patient when taking the image.

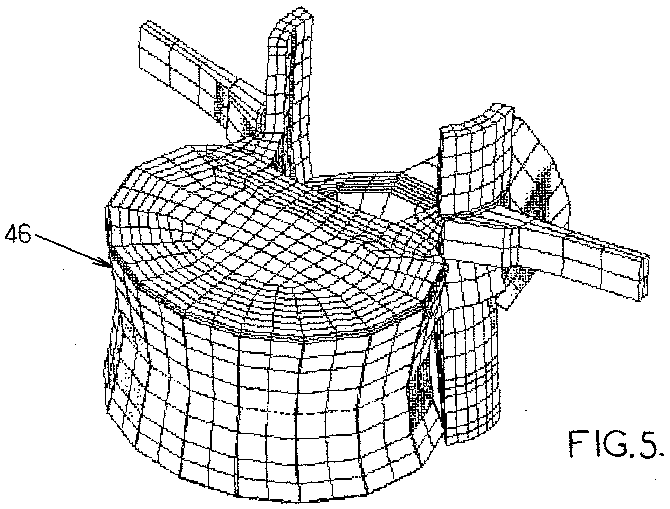

On Fig. 5, the patient specific resulting mesh is represented very schematically.

In a further step, patient-specific bone mechanical properties (e.g. Young's modulus, maximum compressive or tensile strength) are input into the model. Further parameters, such as those related to the anisotropy of the bone material could also be defined. These parameters could be defined individually for each element of the mesh or for parts of the model having, in average, the same mechanical behaviour.

The mechanical property knowledge database 47 is used to correlate the information provided from the detection data, such as the grey level in the radiographic images, to the mechanical characteristics of the bone.

For example, for each surfacic element of the obtained geometric model, a thickness value and a value of the Young's modulus are defined from the mechanical characteristics knowledge base 47. For example, initially, uniform thickness and Young's modulus data are defined over the model elements, based on average thickness and Young's modulus data provided from the mechanical characteristics knowledge base 47.

In variant embodiments, different initial values could be defined for different anatomical regions such as the anterior, lateral, and posterior vertebral walls, the pedicle, etc....

It should be noted that other mechanical parameters could be defined, such as a single parameter combining thickness and Young's modulus (equivalent homogenized modulus for a given thickness).

The initial mechanical characteristics inserted into the geometric model can be modified to be made patient-specific. For example, if we take into account the fact that the grey level on the image is related both to attenuation coefficient of the tissues (which is also related density and thus to elastic modulus) and cortical thickness. We can simulate virtual X-rays according to the initial attenuation/thickness and we can change those initial parameters until the simulated radiographs are made as similar as possible to the real radiographs. This step will lead to patient-specific mechanical properties.

The above is just an example of how to determine the patient's specific geometry and mechanical characteristics. The mechanical characteristics could be determined simultaneously with the determination of the geometry, by using the mechanical characteristics already during the reconstruction step.

In another embodiment, the cortical bone could also be meshed with volumic elements.

Further, the patient-specific position-specific mechanical loads applied to the bone structure in the position of the patient when taking the acquisition data are estimated. For example, these loads are due to gravity. The patient-specific mechanical loads can be assessed from any suitable kind of mechanical model.

For example, the body is virtually divided into segments, such as the head, each upper limb, the trunk, and each of the lower limbs. The trunk segment could be divided in sub-segments, for example at each vertebral level and at the pelvis level. For each segment or sub-segment, the mass and position of the centre of gravity is determined from the geometry at that level. For example, the knowledge base 43 related to the mass and location of the centre of gravity of the body segments comprise an anthropometric table enabling to estimate, from the local geometry, the mass and the location of the centre of gravity of the studied segment or sub-segment. The local geometry could for example be the bone geometry obtained from the radiographs or from the previously obtained mesh, for example.

In this case, the location of the centre of gravity and the mass of the sub-segment can be statistically determined from the knowledge base 43 based only on the location of the bone in one, or both radiographs or on the 3D mesh.

In another embodiment, the location of the centre of gravity and the mass of the sub-segment can be statistically determined using additional data 39, 40 relating to the external envelope of the patient at the sub-segment.

For example, the knowledge base 43 comprises knowledge enabling to determine, for a given envelope, the location of the centre of gravity and the mass of each corporal sub-segment situated above the bony structure of interest.

In this example, as shown on Fig. 4, parts of the patient's envelope are determined. For example, the back and front outlines 48a, 48b of the skin of the patient are detected on the lateral radiographs, and left and right outlines 48c, 48d on the frontal radiograph. These outlines could be determined, either automatically by image processing, or manually by the user. For example, for each sub-segment, the lateral depth between the front and back envelope is measured as shown by arrow D_{L} on Figure 4, and the width between the left and right envelopes is measured, as shown (for another sub-segment) by arrow W on Fig. 4. The knowledge base 43 comprises a priori knowledge of the average density of the sub-segment which allows calculating the mass and the position of the centre of gravity of this sub-segment, as a function of depth and width. This a priori knowledge is used, together with the patient specific measurements, to determine the patient-specific position of the sub-segment's centre of gravity and its mass.

This average density might not distinguish density values for bone and soft tissue and, in this case, is an overall density for the sub-segment. In a variant embodiment where bone detection data is available for the sub-segment, the stored density might distinguish between average soft tissue density and average bone density.

Although the above embodiments describe using both the width and depth of the sub-segment for determining its mass and the location of its centre of gravity, only one of the width and depth could be used, in a variant embodiment for this determination.

This determination is performed for all the sub-segments and segments above the studied structure, so as to evaluate the location and intensity of the gravitational load on the studied structure, by calculating the barycentre of all the segments above the structure.

There are many other possible embodiments for obtaining the mass and the location of the centre of gravity. For example, the medical imager could comprise an optical acquisition device, such as one or more cameras, the images of which can be determined in the same three-dimensional frame of reference as the one of the geometrical 3D model, of the studied structure so as to reconstruct a full 3D envelope. An embodiment could use the projection of structured light onto the patient, detection of this light by cameras, and the processing of the detected optical images by suitable well-known algorithms.

The envelope data is used to determine, in the imaging position (here : standing), the point of application of the gravitational load of the part of the patient situated above the bone structure of which a 3-dimensional reconstruction has been previously established.

In any of the above embodiments, the total mass 41 of the patient could also be used for the determination of the mass and the location of the centre of gravity of the sub-segment.

In a variant embodiment, the mass and the location of the centre of gravity of the segments under the studied structure could be determined using any of the above methods. Then, the mass and the location of the centre of gravity of the part above the studied structure could be determined therefrom and from the data provided from the force platform 42, by an equilibrium calculation.

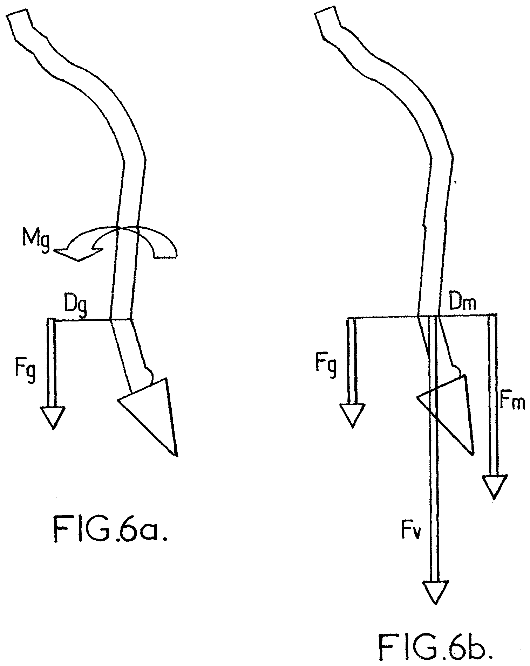

The applied load F_{g} on the bone structure is calculated by the total mass above the bone structure, applied on the centre of mass G of the portions of the patient above the bone structure, and along the vertical direction. It is thus shown on Fig. 6a that the gravity load for a given vertebra is applied at a distance Dg from the vertebra, whereby a moment Mg is applied on the vertebra. As shown on Fig. 6b, this moment can be regulated by a muscular force Fₘ, which is applied at a distance Dm from the vertebra. F_{g} and Fₘ build up the total resultant force Fᵥ applied on the vertebra.

There is a whole range of possibilities to determine the muscular load. One might use a simple muscular model in which only one set of muscles is activated.

More complex models can be used. Taking into account the muscles lever arms (the distance between the gravity line and the bone and the distance between muscle attachments and the bone) it is possible to estimate the loads on the bone. Such mechanical model could be improved using a multiple muscles regulation model. For example a proprioception based regulation model can be proposed to estimate the trunk muscular loads. The muscular model is built as a closed loop regulation system considering the load components within the intervertebral joint as the commands that have to be maintained beyond given thresholds, such as described for example in "A proprioception based regulation model to estimate the trunk muscular forces", Pomero and al., Computer Methods in Biomechanics and Biomedical Engineering, Vol. 7, No 6, December 2004, pages 331-338.

The initial mesh of the generic model could bear information of the nodes or elements which are to be considered as muscle insertion points into the bone. Upon deforming the initial mesh to be rendered patient-specific, these nodes and elements are displaced and/or deformed, which provides with a patient-specific location of muscle insertion.

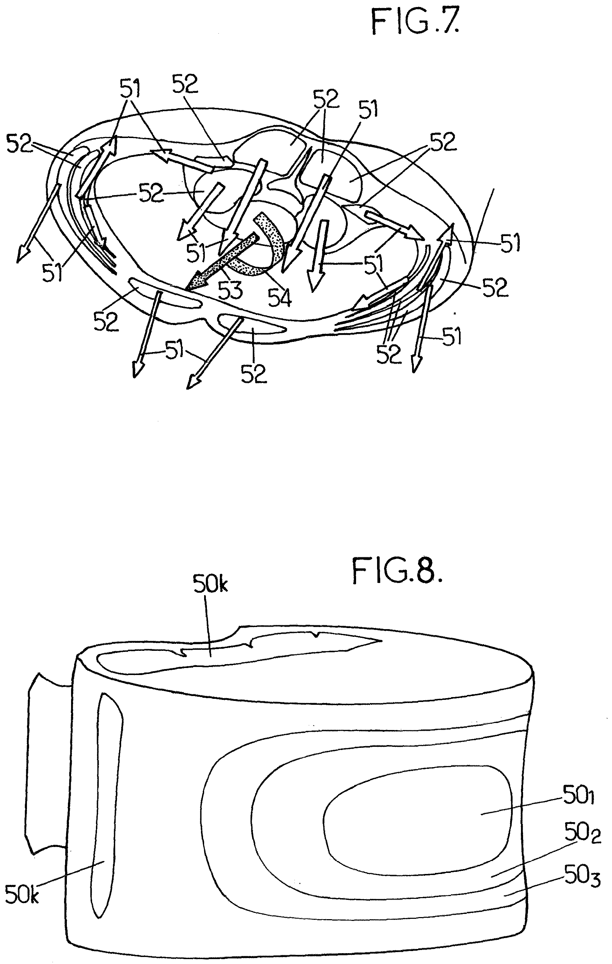

As shown on Fig. 7, arrows 51 represent the intensity of forces exerted by the muscles 52 in the sectional view, as determined from the muscular model. The resulting force 53 and torque 54 at the bone joint are also shown on Fig. 7. Fig. 7 is a purely illustrative view of the muscular loads determined by the muscular model, but is not a view obtained from the medical imager of Figure 1.

The thereby determined loads are expressed in the same frame of reference as the 3D patient-specific mesh with personalised mechanical characteristics.

Finally, the applied loads are input into the finite-element model as shown by arrow Fᵥ on Fig. 6b. The above are just examples of possible patient-specific loads and only examples of how to measure them.

The thus obtained patient-specific and position-specific finite-element model is input in a finite element solver 44, which solves the mechanical problem for the model. The results can be displayed on the screen 19, for example in pseudo-colours, such as schematically illustrated on Fig. 8, on which a level of stress experienced by the patient in the standing position in which he was imaged is represented. The highest stress is found in region 50₁. Decreasing stresses are encountered in regions 50₂, 50₃,..., 50ₖ,.... These results could allow a clinician to visualize on which part of the bone the applied mechanical load has a critical effect in the functional position in which the patient was imaged. Automatic post-processing could be made to determine biomechanically relevant parameters, such as, for example, the highest stress or highest strain encountered in an element, an average stress in a given part of the bone model, a number of contiguous elements having a stress or strain above a predetermined threshold, or any preestablished biomechanical criteria.

These biomechanical parameters could be taken into account by the clinician to determine a risk of fracture for the patient, for example osteoporotic patients and/or to propose a therapy.

The method which was described above was by way of example only. The structure to be studied could be a single bone or a whole osteo-articular structure and could be any part of the body skeleton of a patient, either in a lying or standing position or any other functional position in which gravity induces a critical load on the bone structure (squatting, standing carrying a load,...).

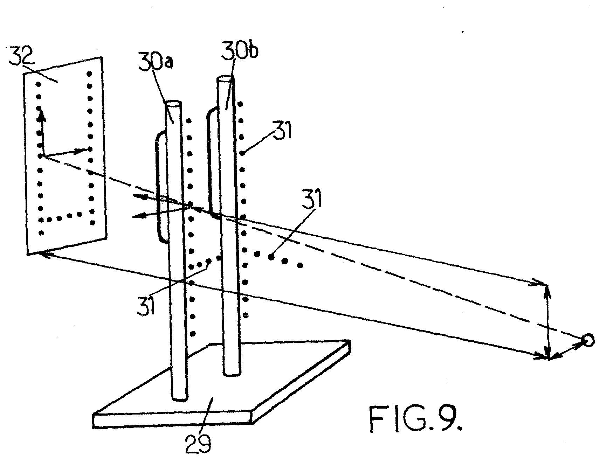

In particular, the acquisition scheme is not limited to the one presented in relation to Fig. 1, wherein a lateral and an antero-posterior images of the structure are obtained simultaneously. One could use other kind of acquisition apparatus, such as the one shown on Fig. 9 comprising a platen 29 and two posts 30a, 30b extending vertically and comprising radio-opaque markers 31 positioned in three-dimensional space so that their detection on the obtained detection data 32 will be used for calibrating the images respective to each other. The platen 29 will be movable relative to the radiological source in order to take a plurality of images of the patient standing on the platen along different orientations. It is even estimated that the present method could be performed using, as detection data, either a pair of non-calibrated radiographs, or a single radiograph.

## Claims

1. A method comprising:
- providing acquisition data (34, 38) of a patient, comprising at least acquisition data (34) of a bone structure of the patient in a given functional position,
- providing a knowledge base (22,43,47), comprising at least data (22) related to structures of the same type as the bone structure of the patient,
- determining, at least from said acquisition data, a patient-specific three-dimensional geometry of the bone structure,
- determining, from said acquisition data and said knowledge base, patient-specific mechanical characteristics of the bone structure,
- determining, at least from the acquisition data, a patient-specific position-specific mechanical load (F) applied to the bone structure in said functional given position,
- assembling the three-dimensional geometry, the mechanical characteristics and the mechanical load into a patient-specific position-specific finite-element model of the bone structure.

2. Method according to claim 1 further comprising one of the following features:
- the knowledge database comprises at least data related to the three-dimensional geometry of structures of the same type as the structure of the patient, and wherein the patient-specific three-dimensional geometry is determined using the knowledge base;
- the knowledge database comprises at least data related to the mechanical characteristics of structures of the same type as the structure of the patient.

3. Method according to any preceding claim wherein the knowledge database comprises at least data related to mass and/or location of a centre of mass of body segments, and wherein the patient-specific position-specific mechanical load is determined using the knowledge base; and optionally wherein acquisition data further comprise acquisition data (39, 40) of at least a part of the external envelope of the patient,
wherein the knowledge base further comprise data (43) related to mass and/or location of a centre of mass correlated to external envelopes.

4. A method according to any preceding claim, wherein providing acquisition data further comprises providing the weight of the patient.

5. A method according to any preceding claim, wherein the mechanical load (F) comprises the load of the mass of the patient situated above the bone structure in said functional position; and optionally wherein the mechanical load further comprises a muscular load applied to the bone structure.

6. A method according to any preceding claim further comprising at least one of the following features:
- acquisition data of the bone structure comprises at least one bi-dimensional image (35) of the bone structure, and wherein the knowledge base comprises at least data (47) related to the mechanical characteristics of bone structures of the same type as the bone structure of the patient;
- acquisition data of the bone structure comprises at least one bi-dimensional image (35) of the bone structure, and the patient-specific three-dimensional geometry is obtained by fitting a generic three-dimensional mesh (20) of the bone structure to the image, said 3D mesh comprising at least one 3D surface.

7. A method according to claim 6, and further comprising one or more of the following features:
- acquisition data of the bone structure comprises at least two bi-dimensional images (35, 36) of the bone structure taken along two non-parallel directions, and referenced in the same reference frame;
- the images are X-ray images.

8. A method according to any preceding claim, further comprising a step of placing the patient in the given functional position in a medical imager, and of acquiring at least said acquisition data of the bony structure; and optionally further comprising a step of detecting a part of the external envelope (48a) of the patient in said given functional position.

9. A method according to any preceding claim, further comprising applying a finite-element-method solver (44) to the patient-specific position-specific model; and optionally comprising determining with the finite-element-method solver (44) a biomechanical criteria of the bone structure.

10. Computer program product comprising instructions for causing a programmable unit to perform the method of any of the preceding claims when executed on said programmable unit.

11. A system comprising:
- a medical imager (1) adapted to provide acquisition data of a patient, comprising at least acquisition data of a bone structure of the patient in a given functional position,
- a knowledge base (22, 43, 47), comprising at least data related to structures of the same type as the structure of the patient,
- a computerized unit (37) adapted to determine, from said acquisition data a patient-specific three-dimensional geometry of the bone structure,
wherein the computerized unit (37) is further adapted to determine from the acquisition data and said knowledge base, a patient-specific mechanical characteristics of the bone structure,
wherein the computerized unit (37) is further adapted to determine, at least from the acquisition data a patient-specific position-specific mechanical load applied to the bone structure in said given functional position,
wherein the computerized unit (37) is further adapted to assemble the three-dimensional geometry, the mechanical characteristics and the mechanical load into a patient-specific position-specific finite-element model of the bone structure.

12. A system according to claim 11 further comprising a finite-element-method solver (44) adapted to be applied to the patient-specific position-specific finite-element model.

13. A system according to claim 11 or 12 wherein the imager comprises at least a first X-ray source (5), a first X-ray detector (6), and a patient-receiving space adapted to receive the patient in standing position between the first source and the first detector, said detector being adapted to detect said acquisition data of the bone structure of the patient; and optionally comprising at least one of the following features:
- the first X-ray source and the first X-ray detector are aligned along a first direction, the imager further comprising a second X-ray source (11) and a second X-ray detector (12) aligned along a second direction not parallel with the first direction;
- the system comprises a displacement device adapted for moving said sources and detectors along a third direction in order to scan the patient.

14. A system according to any of claims 11 to 13, wherein the medical imager further comprises a force platform (42) adapted to detect the gravity axis passing through the patient.

15. A system according to any of claims 11 to 13, wherein the medical imager further comprises at least one camera for detecting at least part of the external envelope of the patient.

## Patentansprüche

1. Verfahren, umfassend:
- Bereitstellen von Aufnahmedaten (34, 38) eines Patienten, umfassend wenigstens Aufnahmedaten (34) einer Knochenstruktur des Patienten in einer gegebenen funktionellen Position,
- Bereitstellen einer Wissensbank (22, 43, 47), umfassend wenigstens Daten (22) bezüglich Strukturen des gleichen Typs wie die Knochenstruktur des Patienten,
- Bestimmen einer Patienten-spezifischen dreidimensionalen Geometrie der Knochenstruktur wenigstens von den Aufnahmedaten,
- Bestimmen von Patienten-spezifischen mechanischen Charakteristiken der Knochenstruktur von den Aufnahmedaten und der Wissensbank,
- Bestimmen einer Patienten-spezifischen Position-spezifischen mechanischen Belastung (F) wenigstens von den Aufnahmedaten, welche auf die Knochenstruktur in der funktionellen gegebenen Position ausgeübt wird,
- Anordnen der dreidimensionalen Geometrie, der mechanischen Charakteristiken und der mechanischen Belastung in einem Patienten-spezifischen Position-spezifischen Finite-Element-Modell der Knochenstruktur.

2. Verfahren nach Anspruch 1, ferner umfassend eines der folgenden Merkmale:
- die Wissensbank umfasst wenigstens Daten bezüglich der dreidimensionalen Geometrie von Strukturen des gleichen Typs wie die Struktur des Patienten, und wobei die Patienten-spezifische dreidimensionale Geometrie unter Verwendung der Wissensbank bestimmt wird;
- die Wissensdatenbank umfasst wenigstens Daten bezüglich der mechanischen Charakteristiken von Strukturen des gleichen Typs wie die Struktur des Patienten.

3. Verfahren nach einem vorhergehenden Anspruch, wobei die Wissensdatenbank wenigstens Daten bezüglich einer Masse und/oder einer Stelle eines Zentrums einer Masse von Körpersegmenten umfasst, und wobei die Patienten-spezifische Position-spezifische mechanische Belastung unter Verwendung der Wissensbank bestimmt wird; und wobei optional Aufnahmedaten ferner Aufnahmedaten (39, 40) wenigstens eines Teils der externen Hülle des Patienten umfassen,
wobei die Wissensbank ferner Daten (43) bezüglich einer Masse und/oder einer Stelle eines Zentrums einer Masse umfasst, korreliert zu externen Hüllen.

4. Verfahren nach einem vorhergehenden Anspruch, wobei das Bereitstellen von Aufnahmedaten ferner ein Bereitstellen des Gewichts des Patienten umfasst.

5. Verfahren nach einem vorhergehenden Anspruch, wobei die mechanische Belastung (F) die Belastung der Masse des Patienten umfasst, welche über der Knochenstruktur in der funktionellen Position angeordnet ist; und wobei optional die mechanische Belastung ferner eine muskuläre Belastung umfasst, welche auf die Knochenstruktur ausgeübt wird.

6. Verfahren nach einem vorhergehenden Anspruch, ferner umfassend wenigstens eines der folgenden Merkmale:
- Aufnahmedaten der Knochenstruktur umfassen wenigstens ein bidimensionales Bild (35) der Knochenstruktur, und wobei die Wissensbank wenigstens Daten (47) bezüglich den mechanischen Charakteristiken der Knochenstrukturen des gleichen Typs wie die Knochenstrukturen des Patienten umfasst;
- Aufnahmedaten der Knochenstruktur umfassen wenigstens ein bidimensionales Bild (35) der Knochenstruktur, und die Patienten-spezifische dreidimensionale Geometrie wird durch Einpassen eines generischen dreidimensionalen Netzes (20) der Knochenstruktur auf das Bild erhalten, wobei das 3-D-Netz wenigstens eine 3-D-Fläche umfasst.

7. Verfahren nach Anspruch 6, ferner umfassend eines oder mehrere der folgenden Merkmale:
- Aufnahmedaten der Knochenstruktur umfassen wenigstens zwei bidimensionale Bilder (35, 36) der Knochenstruktur, welche entlang zwei nichtparallelen Richtungen genommen worden sind, und in dem gleichen Referenz-Rahmen referenziert werden;
- die Bilder sind Röntgenbilder.

8. Verfahren nach einem vorhergehenden Anspruch, ferner umfassend einen Schritt eines Platzierens des Patienten in der gegebenen funktionellen Position in einer medizinischen Bildgebungseinheit und eines Aufnehmens wenigstens der Aufnahmedaten der knöchernen Struktur; und ferner optional umfassend einen Schritt eines Detektierens eines Teils der externen Hülle (48a) des Patienten in der gegebenen funktionellen Position.

9. Verfahren nach einem vorhergehenden Anspruch, ferner umfassend ein Ausüben einer Finite-Element-Verfahren-Auflösungseinheit (44) auf das Patienten-spezifische Position-spezifische Modell; und optional umfassend ein Bestimmen mit der Finite-Element-Verfahren-Auflösungseinheit (44) eines biomechanischen Kriteriums der Knochenstruktur.

10. Computerprogramm-Produkt, umfassend Anweisungen, um eine programmierbare Einheit zu veranlassen, das Verfahren nach einem der vorhergehenden Ansprüche durchzuführen, wenn auf der programmierbaren Einheit ausgeführt.

11. System, umfassend:
- eine medizinische Bildgebungseinheit (1), welche dazu eingerichtet ist, Aufnahmedaten eines Patienten bereitzustellen, umfassend wenigstens Aufnahmedaten einer Knochenstruktur des Patienten in einer gegebenen funktionellen Position,
- eine Wissensbank (22, 43, 47), umfassend wenigstens Daten (22) bezüglich Strukturen des gleichen Typs wie die Knochenstruktur des Patienten,
- eine computerisierte Vorrichtung (37), welche dazu eingerichtet ist, eine Patienten-spezifische dreidimensionale Geometrie der Knochenstruktur wenigstens von den Aufnahmedaten zu bestimmen,
wobei die computerisierte Vorrichtung (37) ferner dazu eingerichtet ist, von den Aufnahmedaten und der Wissensbank Patienten-spezifische mechanische Charakteristiken der Knochenstruktur zu bestimmen,
wobei die computerisierte Vorrichtung (37) ferner dazu eingerichtet ist, eine Patienten-spezifische Position-spezifische mechanische Belastung (F) wenigstens von den Aufnahmedaten, welche auf die Knochenstruktur in der funktionellen gegebenen Position ausgeübt wird, zu bestimmen,
wobei die computerisierte Vorrichtung (37) ferner dazu eingerichtet ist, die dreidimensionale Geometrie, die mechanischen Charakteristiken und die mechanische Belastung in einem Patienten-spezifischen Position-spezifischen Finite-Element-Modell der Knochenstruktur anzuordnen.

12. System nach Anspruch 11, ferner umfassend eine Finite-Element-Verfahren-Auflösungseinheit (44), welche dazu eingerichtet ist, auf das Patienten-spezifische Position-spezifische Finite-Element-Modell angewendet zu werden.

13. System nach Anspruch 11 oder 12, wobei die Bildgebungseinheit wenigstens eine erste Röntgenstrahlen-Quelle (5), einen ersten Röntgenstrahlen-Detektor (6) und einen einen Patienten aufnehmenden Raum umfasst, welcher dazu eingerichtet ist, den Patienten in einer stehenden Position zwischen der ersten Quelle und dem ersten Detektor aufzunehmen, wobei der Detektor dazu eingerichtet ist, die Aufnahmedaten der Knochenstruktur des Patienten zu detektieren; und optional umfassend wenigstens eines der folgenden Merkmale:
- die erste Röntgenstrahlen-Quelle und der erste Röntgenstrahlen-Detektor sind entlang einer ersten Richtung ausgerichtet, wobei die Bildgebungseinheit ferner eine zweite Röntgenstrahlen-Quelle (11) und einen zweiten Röntgenstrahlen-Detektor (12) umfasst, welche entlang einer zweiten Richtung ausgerichtet sind, welche zu der ersten Richtung nicht parallel ist;
- das System umfasst eine Verlagerungsvorrichtung, welche dazu eingerichtet ist, die Quellen und Detektoren entlang einer dritten Richtung zu bewegen, um den Patienten zu scannen.

14. System nach einem der Ansprüche 11 bis 13, wobei die medizinische Bildgebungseinheit ferner eine Kraft-Plattform (42) umfasst, welche dazu eingerichtet ist, die Schwerkraftachse zu detektieren, welche durch den Patienten hindurch verläuft.

15. System nach einem der Ansprüche 11 bis 13, wobei die medizinische Bildgebungseinheit ferner wenigstens eine Kamera zum Detektieren wenigstens eines Teils der äußeren Hülle des Patienten umfasst.

## Revendications

1. Procédé comprenant :
- la fourniture de données d'acquisition (34, 38) d'un patient, comprenant au moins des données d'acquisition (34) d'une structure osseuse du patient dans une position fonctionnelle donnée,
- la fourniture d'une base de connaissances (22, 43, 47), comprenant au moins des données (22) relatives à des structures du même type que la structure osseuse du patient,
- la détermination, au moins à partir desdites données d'acquisition, d'une géométrie tridimensionnelle spécifique au patient de la structure osseuse,
- la détermination, à partir desdites données d'acquisition et de ladite base de connaissances, de caractéristiques mécaniques spécifiques au patient de la structure osseuse,
- la détermination, au moins à partir des données d'acquisition, d'une charge mécanique (F) spécifique au patient et spécifique à la position appliquée à la structure osseuse dans ladite position fonctionnelle donnée,
- l'assemblage de la géométrie tridimensionnelle, des caractéristiques mécaniques et de la charge mécanique dans un modèle d'élément fini de la structure osseuse spécifique au patient et spécifique à la position.

2. Procédé selon la revendication 1 comprenant en outre l'une des caractéristiques suivantes :
- la base de connaissances comprend au moins des données relatives à la géométrie tridimensionnelle de structures du même type que la structure du patient, et dans lequel la géométrie tridimensionnelle spécifique au patient est déterminée à l'aide de la base de connaissances ;
- la base de connaissances comprend au moins des données relatives aux caractéristiques mécaniques de structures du même type que la structure du patient.

3. Procédé selon une quelconque revendication précédente dans lequel la base de connaissances comprend au moins des données relatives à la masse et/ou à l'emplacement d'un centre de masse de segments corporels, et dans lequel la charge mécanique spécifique au patient et spécifique à la position est déterminée à l'aide de la base de connaissances ; et optionnellement dans lequel les données d'acquisition comprennent en outre des données d'acquisition (39, 40) d'au moins une partie de l'enveloppe externe du patient,
dans lequel la base de connaissances comprend en outre des données (43) relatives à la masse et/ou à l'emplacement d'un centre de masse corrélées aux enveloppes externes.

4. Procédé selon une quelconque revendication précédente, dans lequel la fourniture de données d'acquisition comprend en outre la fourniture du poids du patient.

5. Procédé selon une quelconque revendication précédente, dans lequel la charge mécanique (F) comprend la charge de la masse du patient située au-dessus de la structure osseuse dans ladite position fonctionnelle, et optionnellement dans lequel la charge mécanique comprend en outre une charge musculaire appliquée à la structure osseuse.

6. Procédé selon une quelconque revendication précédente, comprenant en outre au moins l'une des caractéristiques suivantes :
- les données d'acquisition de la structure osseuse comprennent au moins une image bidimensionnelle (35) de la structure osseuse, et dans lequel la base de connaissances comprend au moins des données (47) relatives aux caractéristiques mécaniques de structures osseuses du même type que la structure osseuse du patient ;
- les données d'acquisition de la structure osseuse comprennent au moins une image bidimensionnelle (35) de la structure osseuse, et la géométrie tridimensionnelle spécifique au patient est obtenue en appliquant un maillage tridimensionnel générique (20) de la structure osseuse à l'image, ledit maillage 3D comprenant au moins une surface 3D.

7. Procédé selon la revendication 6, et comprenant en outre l'une ou plusieurs des caractéristiques suivantes :
- les données d'acquisition de la structure osseuse comprennent au moins deux images bidimensionnelles (35, 36) de la structure osseuse prises le long de deux directions non parallèles, et référencées dans le même cadre de référence ;
- les images sont des images par rayons X.

8. Procédé selon une quelconque revendication précédente, comprenant en outre une étape consistant à placer le patient dans la position fonctionnelle donnée dans un appareil d'imagerie médicale, et à acquérir au moins lesdites données d'acquisition de la structure osseuse ; et optionnellement comprenant en outre une étape consistant à détecter une partie de l'enveloppe externe (48a) du patient dans ladite position fonctionnelle donnée.

9. Procédé selon une quelconque revendication précédente, comprenant en outre l'application d'un solveur utilisant la méthode des éléments finis (44) au modèle spécifique au patient et spécifique à la position ; et optionnellement comprenant la détermination à l'aide du solveur utilisant la méthode des éléments finis (44) d'un critère biomécanique de la structure osseuse.

10. Produit de programme informatique comprenant des instructions pour amener une unité programmable à réaliser le procédé selon l'une quelconque des revendications précédentes lorsqu'elles sont exécutées sur ladite unité programmable.

11. Système comprenant :
- un appareil d'imagerie médicale (1) adapté pour fournir des données d'acquisition d'un patient, comprenant au moins des données d'acquisition d'une structure osseuse du patient dans une position fonctionnelle donnée,
- une base de connaissances (22, 43, 47), comprenant au moins des données relatives à des structures du même type que la structure du patient,
- une unité informatisée (37) adaptée pour déterminer, à partir desdites données d'acquisition, une géométrie tridimensionnelle spécifique au patient de la structure osseuse,
dans lequel l'unité informatisée (37) et en outre adaptée pour déterminer, à partir des données d'acquisition et de ladite base de connaissances, des caractéristiques mécaniques spécifiques au patient de la structure osseuse,
dans lequel l'unité informatisée (37) est en outre adaptée pour déterminer, au moins à partir des données d'acquisition, une charge mécanique spécifique au patient et spécifique à la position appliquée à la structure osseuse dans ladite position fonctionnelle donnée,
dans lequel l'unité informatisée (37) est en outre adaptée pour assembler la géométrie tridimensionnelle, les caractéristiques mécaniques et la charge mécanique dans un modèle d'élément fini de la structure osseuse spécifique au patient et spécifique à la position.

12. Système selon la revendication 11 comprenant en outre un solveur utilisant la méthode des éléments finis (44) adapté pour être appliqué au modèle d'élément fini spécifique au patient et spécifique à la position.

13. Système selon la revendication 11 ou 12 dans lequel l'appareil d'imagerie comprend au moins une première source de rayons X (5), un premier détecteur de rayons X (6), et un espace recevant un patient adapté pour recevoir le patient en position debout entre la première source et le premier détecteur, ledit détecteur étant adapté pour détecter lesdites données d'acquisition de la structure osseuse du patient ; et optionnellement comprenant au moins l'une des caractéristiques suivantes :
- la première source de rayons X et le premier détecteur de rayons X sont alignés le long d'une première direction, l'appareil d'imagerie comprenant en outre une deuxième source de rayons X (11) et un deuxième détecteur de rayons X (12) alignés le long d'une deuxième direction non parallèle à la première direction ;
- le système comprend un dispositif de déplacement adapté pour déplacer lesdites sources et lesdits détecteurs le long d'une troisième direction afin de scanner le patient.

14. Système selon l'une quelconque des revendications 11 à 13, dans lequel l'appareil d'imagerie médicale comprend en outre une plateforme de force (42) adaptée pour détecter l'axe de gravité traversant le patient.

15. Système selon l'une quelconque des revendications 11 à 13, dans lequel l'appareil d'imagerie médicale comprend en outre au moins une caméra pour détecter au moins une partie de l'enveloppe externe du patient.
